# EUROPEAN PATENT SPECIFICATION

(11) **EP 2 393 778 B1**
(45) Date of publication and mention of the grant of the patent: **09.04.2014**
(21) Application number: 10707659.8
(22) Date of filing: 05.02.2010
(51) Int. Cl.: C07D 209/34, A61K 31/4045, A61P 25/18

(54) **Optically active 3-[(phenylpiperazin-1-yl)alkyl]-3- alkyl-oxindole derivatives having CNS activity**
OPTISCH AKTIVE 3-[(PHENYLPIPERAZIN-1-YL)ALKYL]-3-ALKYL-OXINDOLDERIVATE MIT ZNS-AKTIVITÄT
Dérivés de 3 [(phénylpipérazine-1-yl)alkyl]-3-alkyl-oxindole optiquement actifs possédant une activité sur le SNC

(30) Priority: 06.02.2009 HU 0900071
(43) Date of publication of application: 14.12.2011
(73) Proprietor: Egis Gyógyszergyár Nyilvánosan Müködö Részvénytársaság, 1106 Budapest (HU)
(72) Inventor: VOLK, Balázs, H-1106 Budapest (HU); BARKÓCZY, József, H-1016 Budapest (HU); GACSALYI, István, H -1201 Budapest (HU); FOGASSY, Elemér, H -2030 Érd (HU); SCHINDLER, József, H - 2151 Fót (HU); GIGLER, Gábor, H-1119 Budapest (HU); KOMPAGNE, Hajnalka, H -1221 Budapest (HU); NAGYNÉ GYÖNÖS, Ildikó, H -1192 Budapest (HU); PALLAGI, Katalin, H -1054 Budapest (HU); PORCS-MAKKAY, Márta, H -2013 Pomáz (HU); SZÉNÁSI, Gábor, H-2096 Üröm (HU); MEZEI, Tibor, H -1221 Budapest (HU); LUKÁCS, Gyula, H - 1163 Budapest (HU); LÉVAY, György, H - 2092 Budakeszi (HU); EGYED, András, H - 1145 Budapest (HU); HÁRSING, László Gábor, H - 1071 Budapest (HU)
(74) Representative: Stolmár & Partner
(86) International application number: PCT/HU2010/000014
(87) International publication number: WO 2010/089616

(56) References cited:
- WO-A2-2005/109987

## Description

### Technical field of the invention

The present invention relates to the enantiomers of 5,7-dichloro-3-{4-[4-(4-chlorophenyl)-piperazin-1-yl]butyl}-3-ethyl-1,3-dihydro-2*H*-indol-2-one of the Formula (II) 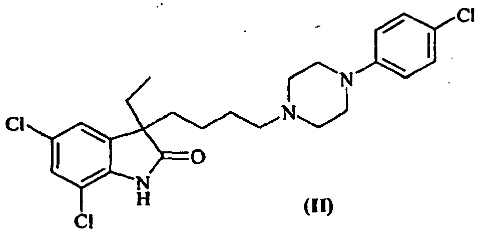 pharmaceutically acceptable salts thereof, process for the preparation thereof, medicinal products containing said enantiomers and said enantiomers and their pharmaceutically acceptable salts for use in the treatment of the disorders of the central nervous system.

### Background of the invention

From the end of the twentieth century, the civilized man is confronted with an increasing amount of information resulting from the exploding pace of technical and social development This process results in a continous change in the adaptation pattern of man to his environment. The continously changing enviroment renders the adjustment to the environment more and more difficult, which results in adaptation disorders. Such adaptation disorders are manifested in the form of mental or psychosomatic diseases, i.e. anxiety, stress, depression, schizofrenia, gastric ulcer, hypertonia etc.

The presently established therapeutic procedures used in the treatment of the above-mentioned disorders involve the administration of medicaments to the patient. One type of the active ingredients used in said medicaments exert their effect by acting through the benzodiazepine system (e.g. diazepam). Other types of active ingredients useful for the treatment or prevention of mental or psychosomatic disorders are effective by influencing the central serotonin 5-HT_{1A} receptors (e.g. buspiron). When a patient is diagnosed with a psychosomatic disorder, the anxiolytic therapy is often supplemented with an antihypertensive (drugs acting through the α₁ or α₂ receptors) or an antiulcer (e.g. H₂ receptor antagonist) active ingredient.

Benzodiazepine-type anxiolytics, however, exhibit several undesirable side effects, including sedation, reduction of concentration, decrease in muscular tension. Such undesirable side effects significantly limit the application area of said drugs and can result in significant negative impact on the life quality of the patient.

A further disadvantage of anxiolytics effective through the serotonin system (i.e. buspirone, SSRIs) resides in the fact that said drugs become clinically effective only after a ten to fourteen-day period of administration. It has been furthermore observed that said drugs exhibit anxiogenic (i.e. anxiety-inducing) effect in the very first period of administration. Said side effects render the cooperation of the patient and the physician difficult, since initially patients percieve that deterioration in their condition is due to the administration of the medicine.

Another problem of the modem society is the rapid ageing of the population. Due to the development of modem medicinal science, the life expectancy has been increasing significantly, which is closely followed by the sudden increase in the incidence of diseases appearing in old age, especially those diseases which affect mental abilities. There exists a social demand for methods and medicinal products suitable for the treatment of Alzheimer-disease, vascular dementias and senile dementia.

Furthermore, there is an increased demand for new, highly effective medicaments which can be used more effectively in the therapy than the medicinal products currently in use. The subject of the present invention is the discovery of such new, pharmaceutically active compounds.

The racemic compound 5,7-dichloro-3-{4-[4-(4-chlorophenyl)-piperazin-1-yl]butyl}-3-ethyl-1,3-dihydro-2*H*-indol-2-one of the Formula (II) has been disclosed for the first time in Published International Patent Application No. WO 2005/109987.

### Summary of the invention

3,3-dialkyl-substituted indol-2-one derivatives disclosed in International Patent Application No. WO 2005/109987 possess significant anxiolytic activity. Said compounds bind to the 5-HT_{2C} receptor and α₁-receptors and exert dopamine release. At the same time, said compounds do not bind to the 5-HT_{1A} receptors. This activity profile has the advantage that the above-mentioned compounds are devoid of undesirable side effects characteristic to those compounds which bind to the 5-HT_{1A} receptor.

The present invention is based on the surprising recognition that the enantiomers of the Formula (I) differ significantly from each other and the racemic compound of the Formula (II) with regard to their pharmacological effects and receptor-binding profile.

### Detailed description of the invention

According to the first aspect of the present invention, there is provided (+)-5,7-dichloro-3-{4-[4-(4-chlorophenyl)-piperazin-1-yl]butyl}-3-ethyl-1,3-dihydro-2*H*-indol-2-one of the Formula (I) 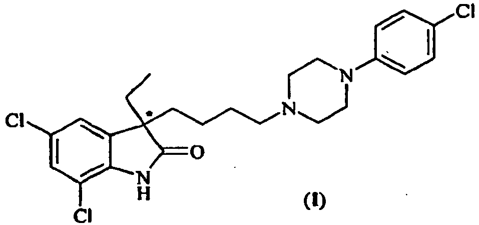 and pharmaceutically acceptable acid addition salts thereof.

According to the second aspect of the present invention, there is provided (-)-5,7-dichloro-3-{4-[4-(4-chlorophenyl)-piperazin-1-yl]-butyl}-3-ethyl-1,3-dihydro-2*H*-indol-2-one of the Formula (I) 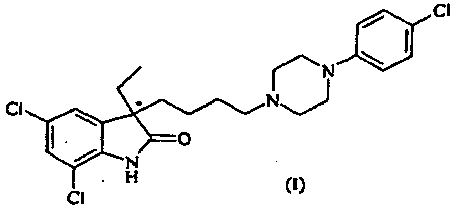 and pharmaceutically acceptable salts thereof.

According to a further aspect of the present invention, there is provided a process for the preparation of the enantiomers of the Formula (I) and salts thereof. As starting compound of the process, racemic 5,7-dichloro-3-{4-[4-(4-chlorophenyl)-piperazin-1-yl]butyl}-3-ethyl-1,3-dihydro-2*H*-indol-2-one of the Formula (II) is used. Said compound can be prepared according to the disclosure of International Patent Application No. WO 2005/109987.

Enantiomers of the Formula (I) can be prepared from the racemate of the Formula (II) by resolving the racemate with an optically active acid. Surprisingly, we have found that although there are no known processes according to the state of the art which could be used for oxindole derivatives wherein the basic nitrogen atom is separated by four carbon atoms from the oxindole group, the enantiomers of the starting compound of the Formula (II) can be resolved in good yield, despite of the fact that the distance of the chiral center and the basic nitrogen atom is much greater than that in the resolving processes known from the prior art.

The resolution is carried out in a dipolar aprotic or protic solvent in the presence of an optically active acid. As a solvent, an alkanol comprising 1 to 4 carbon atoms, water or mixtures thereof, preferably the mixture of ethanol and water can be used. As resolving acid, an optically active tartaric acid derivative, advantageously optically active dibenzoyl-tartartic acid or optically active ditoluyl-tartaric acid can be used.

The compounds of the Formula (I) thus obtained can thereafter be converted into a pharmaceutically acceptable acid addition salt or the base can be set free from an acid addition salt.

The pharmacological activity of the compounds of the Formula (I) according to the present invention has been tested in receptor-binding assays, Vogel's drinking conflict model in rats, Porsolt's test in mice and permanent focal cerebral ischemia model in mice.

In the receptor-binding assays, brain tissues from male Wistar rats weighing 120-200 g or cloned human 5-HT₆ or D₄ receptors were used. The protein contents of the membrane preparates were determined according to Lowry (Lowry O. H., Rosenbrough N. J., Farr A. L., Randall R. J., Protein measurement with the Folin-Phenol reagents. J Biol Chem, 193: 265-275 (1951).

The basic information with regard the receptor-binding assay is disclosed in Table 1. The binding assays were carried out according to the method of Leysen (Leysen J. E, Niemegeers C. J. E, Van Nueten J. M., Laduron P. M.: [3H]Ketanserin (R41468), a selective [3H]ligand for Serotonine2 receptor binding. Mol Pharmacol, 21: 301-314 (1981)). The tested compound was deemed to be effective in the case when the Kᵢ value was smaller than 100 nM. The results are summarized in Table 2.

**Table 1**

| **Receptor** | **Ligand** | **Tissue** | **NSL* (conc.)** | **Reference** |
|---|---|---|---|---|
| 5-HT₆ | ³H-LSD (2.7 nM) | HEK 293 cells | 5-HT creatinine sulphate (100.0 µM) | According to the instructions provided with the set * |
| 5-HT_{2A} | ³H-ketanserine 1.0 nM | Frontal cortex | cyproheptadine (10.0 µM) | Leysen, 1981 |
| D₄ | ³H-YM-09151-2 (0.28 nM) | SF9 cells | clozapine (10 µM) | According to the instructions provided with the set * |

| | | | | |
|---|---|---|---|---|
| *Receptor Biology Inc., Boston, USA ** BioSignal Packard Inc., Montreal, Kanada | | | | |

**Table 2**

| **Receptor** | **Compound of the Formula (II) Kᵢ (nM)** | **(+)-enantiomer of the Formula (I) Kᵢ (nM)** | **(-)-enantiomer of the Formula (I) kᵢ (nM)** |
|---|---|---|---|
| 5-HT₆ | 9 | 11 | 1000 |
| 5-HT_{2A} | 50 | >200 | 42 |
| D₄ | >100 | 35 | 13 |

Vogel's drinking conflict model was used for establishing the anxiolytic effect (Vogel at el., Psychopharmacologia, 21, 1, 1971). Male Wistar rats weighing 200 to 220 grams were restrained from drinking for 48 hours and deprived from feed for 24 hours prior to the experiment. The test substances or the vehicle were administered to the animals in a dose of 5.0-10.0-20.0 mg/kg intraperitoneally 30 minutes before the test. The test chamber was provided with a drinking tube which allowed the animals to drink. However, after each twentieth licking; the apparatus delivered a 0.7 mA electric shock to the animal. During the five-minute measurement period, the number of electric shocks was registered which the animals tolerated to appease their thirst. The effect of the test substance was expressed in the percentage increase in the number of tolerated electric shocks. The minimum effective dose (MED) was determined for each compound. The results are summarized in Table 3.

**Table 3**

| **ModelI** | **Test substance, dose** | | |
|---|---|---|---|
| | **Compound of the Formula (II)** | **(+)-enantiomer of the Formula (I)** | **(-)-enantiomer of the Formula (I)** |
| Vogel-test (model for anxiolytic effect, mg/kg ip., MED) | 5 | 10 | No effect observed |
| Porsolt-test (model for antidepressant effect, mg/kg ip., MED) | 3 | No effect observed | No effect observed |
| Permanent cerebral ischemia model (model for neuroprotective effect, mg/kg ip., MED) | 0.1 | No effect observed | 3.0 |

| | | | |
|---|---|---|---|
| MED= minimum dose resulting in statistically significant effect | | | |

The antidepressant effect of the compounds of the Formula (I) has been established in Porsolt's test in male DBA mice weighing 20 to 25 grams. The swimming experiment was carried out in cylinders of 17.7 cm height and 12 cm diameter (Porsolt, R.D.: Behavioral despair. In: Enna, S.J., Malick, J.B., and Richelson, E. (ends.): " Antidepressants: Neurochemical, Behavioral, and Clinical Prespectives." New York: Raven Press, 1981.pp. 121-139). In each experiment, four cylinders were used simultaneously. The animals were treated 30 minutes before the test with the test substances or with the vehicle in the dose of 1-3-10 mg/kg i.p. Animals were allowed to stay in the cylinder for six minutes. In the first two minutes, no measurement was made since this period was considered as the adaptation period. In the following four minutes (minutes 3 to 6 of the experiment) the time spent in immobile state was determined. The immobile time was registered for each animal with a precision of one second. The average was calculated for each group and the percentage effect was calculated for each treated group with reference for the control group. The statistical significance of the differences between groups was analyzed by Duncan-test followed by one-way variance analysis.

On the basis of the statistical significance determined for the difference of each dose from the control, MED (minimum effective dose) values were calculated. This is the smallest dose which resulted in a significant (p<0.05) effect. A significant decrease of time spent in immobility was interpreted as an antidepressant effect. The results are summarized in Table 3.

The neuroprotective effect of the compounds of the Formula (I) was tested in the permanent focal cerebral ischemia model according to the modified method of Karkoutly and coworkers (Karkoutly, C., Backhauss, C., Nuglisch, J., Krieglstein, J.: The measurement of the infarcted area after middle cerebral artery occlusion in the mouse: a screening model. In Krieglstein, J., Oberpichler, H. ed. Pharmacology of Cerebral Ischemia 1990. Wissenschaftliche Verlagsgesellschaft mbH Stuttgart. 63-69 (1990).).

Male NMRI mice weighing 30 to 35 grams were anesthetized with 2,2,2-tribromoethanol administered intraperitoneally in the dose of 500 mg/kg (20 ml/kg). During anesthesia, surgery was performed according to the surgical protocol of Welsh and coworkers (Welsh, F. A., Sakamoto, T., McKee, A., Sims, R. E.: Effect of lactacidosis on pyridine nucleotide stability during ischemia in mouse. J. Neurochem. 49: 846-851 (1987)). The distal part of the middle carotid artery (MCA) was occluded with electric cauter. Test compounds were administered intraperitoneally 30 minutes after surgery in the dose of 0.1-0.3-1.0-3.0-10.0 mg/kg). After 48 hours, mice were anesthetised by 120 mg/kg sodium pentobarbital intraperitoneally. The brains were perfused through the left ventricle with 2 ml of 4 % 2,3,5-triphenyl-tetrazolium-chloride (TTC) solution. After one hour, the brains was removed and placed into ice-cool saline for one to two minutes. Subsequently the brains were placed into 8 % aqueous formaldehyde solution. After 24 hours, the area of white lesions was determined by a computerized image analysis software. The statistical evaluation was performed by variance analysis followed by Duncan-test. Results thus obtained are demonstrated in Table 3.

On the basis of the tests described above, we have found that the racemic compound of the Formula (II) exhibited significant affinity towards central 5-HT₆ receptors, similarly to the (+)-enantiomer of the Formula (I). However, we surprisingly found that the (-)-enantiomer of the Formula (I) was not bound to the 5-HT₆ receptors. Unexpectedly, we have also found that the (+)-enantiomer of the Formula (I) did not show any significant affinity to the central 5-HT_{2A} receptors, while at the same time, the racemic compound of the Formula (II) and the (-)-enantiomer was bound to the 5-HT_{2A} receptors with an affinity greater by several orders of magnitude. Furthermore, we surprisingly recognized that the three compounds exhibit significantly different affinity to the central D₄ receptors. We have found that the racemic compound of the Formula (II) did not show any significant binding. In contrary, the two enantiomers of the Formula (I), especially the (-)-enantiomer have significant affinity even at lower concentration range (Table 2).

Besides the difference in the receptor binding profiles, significant difference was experienced in the predicted therapeutical application area of the three substances as well.

Significant effect suitable for prognosing the anxiolytic activity was found in the Vogel's drinking conflict test for 5,7-dichloro-3-{4-[4-(4-chlorophenyl)-piperazin-1-yl]butyl}-3-ethyl-1,3-dihydro-2*H*-indol-2-one of the Formula (II) after administering said compound in the dose of 5 mg/kg intraperitoneally. (+)-enantiomer of the Formula (I) showed a significant anxiolytic effect in the dose of 10 mg/kg. In contrast to the results obtained for the above-mentioned compounds, (-)-5,7-dichloro-3-{4-[4-(4-chlorophenyl)-piperazin-1-yl]butyl}-3-ethyl-1,3-dihydro-2*H*-indol-2-one of the Formula (I) did not show any anxiolytic effect (Table 3).

A significant and unexpected difference was observed among the three compounds in the Porsolt's test in mice. Said method can be used for the prediction of antidepressant effect. The racemic compound of the Formula (II) exhibited a significant, dose-dependent antidepressant activity, while no antidepressant effect was observed for the individual enantiomers (Table 3).

With regard to the neuroprotective effect, which indicates the suitability of the test compound for the treatment of cerebral cell death, a surprising difference was observed among the racemic 5,7-dichloro-3-{4-[4-(4-chlorophenyl)-piperazin-1-yl]butyl}-3-ethyl-1,3-dihydro-2*H*-indol-2-one of the Formula (II) and the two optically active isomers thereof. Cell death resulting from the occlusion of middle cerebral artery was prevented in a very low (0.1 mg/kg i.p.) dose by the racemic compound of the Formula (II). The (+)-enantiomer of the Formula (I) proved to be ineffective in this test. The (-)-enantiomer of the Formula (I) proved to be effective only in a significantly higher dose range (3 mg/kg i.p.) than the racemate (Table 3).

In summary, it was unexpectedly found that the three compounds showed surprisingly different pharmacological and consequently different therapeutical profile. Racemic 5,7-dichloro-3-{4-[4-(4-chlorophenyl)-piperazin-1-yl]butyl}-3-ethyl-1,3-dihydro-2*H*-indol-2-one of the Formula (II) exhibited significant anxiolytic, antidepressant and neuroprotective effect. In contrast to the above findings, the (+)-enantiomer of the Formula (I) showed anxiolytic acitivity only while being ineffective in the animal models suitable for the prediction of antidepressant potential and neuroprotective effect. The (-)-enantiomer of the Formula (I) exhibited exclusively neuroprotective effect.

According to the further aspect of the present invention, there are provided medicaments which comprise an enantiomer of the Formula (I) or a
pharmaceutically acceptable salt thereof alone or in admixture with one or more vehicles or auxiliary agents known from the prior art.

The medicament according to the present invention contains the active ingredient usually in the concentration of 0.1-95 percent by weight, preferable in the concentration of 1 to 50 percent by weight, the most preferably, in the concentration of 5 to 30 percent by weight. Medicaments according to the present invention can be administered orally (e.g. powders, tablets, coated tablets, capsules, microcapsules, dragees, solutions, suspensions or emulsions), parenterally (e.g. in the form of intravenous, intramuscular, subcutaneous or intraperitoneal injections or in the form of an infusion), rectally (e.g. in the form of suppositories), transdermally (e.g. in the form of patches), as implants or locally (e.g. as creams, ointments or patches). Solid, semisolid and liquid medicaments according to the present invention can be prepared according to the processes known per se from the state of the art.

Solid medicaments suitable for oral administration containing a compound of the Formula (I) or a pharmaceutically acceptable salt thereof as pharmaceutically active ingredient can contain vehicles, filling agents (e.g. lactose, glucose, starch, calcium phosphate, microcrystalline cellulose), binding agents (e.g. gelatin, sorbitol, polyvinylpyrrollydone), disintegrants (e.g. croscarmellose, sodium carboxymethyl-cellulose, crospovidone), tabletting aids (e.g. magnesium stearate, talc, polyethyleneglycol, silicic acid, silicon dioxide) or surfactants (e.g. sodium lauryl sulphate).

Liquid medicaments intended for oral administration containing a compound of the Formula (I) or a pharmaceutically acceptable salt thereof as active ingredient can be presented in the form of e.g. solutions, suspensions or emulsions and can contain suspending agents (e.g. gelatin, carboxymethyl cellulose), emulsifying agents (e.g. sorbitan monooleate), solvents (e.g. water, oils, glycerol, propylene glycol, ethanol), pH adjusting agents (e.g. acetate, phosphate, citrate buffers) or stabilizing agents (e.g. methyl-4-hydroxy-benzoate).

Liquid medicaments containing a compound of the Formula (I) or a pharmaceutically acceptable salt thereof suitable for parenteral administration are sterile isotonic solutions, which contain a pH-adjusting agent and a stabilizing agent besides the solvent.

Semisolid medicaments containing a compound of the Formula (I) or a pharmaceutically acceptable salt thereof as active ingredient, e.g. suppositories, contain the active ingredient of the Formula (I) homogeneously dispersed in the base of the suppository (e.g. in polyethylene glycol or cocoa butter).

According to a futher aspect of the present invention, there is provided the use of the compounds of the Formula (I) and pharmaceutically acceptable acid addition salts thereof for the preparation of a medicament suitable for the treatment or prevention of the diseases of the central nervous system including psychosomatic disorder, anxiety, generalized anxiety disorder, panic disorder, compulsive disorder, social phobia, agoraphobia, phobias occuring in specific situations, posttraumatic stress disorder, memory disturbance following traumas, cognitive disorder, sexual disorder originating from the central nervous system origin, depression, schizofrenia, kidney insufficiency, loss of hearing, tinnitus, gastrointestinal disorders and cardiovascular diseases.

Medicaments containing a compound of the Formula (I) or a pharmaceutically acceptable salt thereof as active ingredient can be produced by the methods of pharmaceutical technology known from the state of the art. The active ingredient is admixed with solid or liquid vehicles or auxiliary agents and is converted into a pharmaceutical dosage form. Vehicles and auxiliary agents and the processes suitable for the manufacture of the medicament are known from the art (Remington's Pharmaceutical Sciences, Edition 18, Mack Publishing Co., Easton, USA, 1990).

Medicaments according to the present invention containing a compound of the Formula (I) or a pharmaceutically acceptable salt thereof as active ingredient contain the active ingredient in the form of dosage units.

The daily dose of a compound of the Formula (I) or a pharmaceutically acceptable salt thereof is 0.1-1000 mg/kg body weight for adults. The daily dose can be administered in one or more portion. The actual dose depends on several factors and the prescription of the dose is the responsibility of a physician.

According to a further aspect of the present invention, there is provided compounds of the Formula (I) or a pharmaceutically acceptable salt thereof for use in the treatment or prevention of the diseases of the central nervous system including psychosomatic disorders, anxiety disorders, generalized anxiety disorder, panic disorder, compulsive disorder, social phobia, agoraphobia, phobias related to specific situations, stress disorder, posttraumatic stress disorder, posttraumatic cognitive disorder, cognitive disorder, sexual disorder originating from the central nervous system, kidney insufficiency, loss of hearing, depression, schizofrenia, mental decline due to cerebral cell death, Alzheimer-disease, stroke, dementia, gastrointestinal disorders or cardiovascular disorders including hypertonia, damage of the hearing organ due to pharmacotherapy or tinnitus.

The invention is demonstrated by the following examples without limiting the invention by any way.

### Example 2

### 5,7-Dichloro-3-(4-chlorobutyl)-3-ethyl-1,3-dihydro-2H-indol-2-one

3-(4-chlorobutyl)-3-ethyl-1,3-dihydro-2*H*-indol-2-one (10.06 g; 40 mmol) are dissolved in 80 ml of glacial acetic acid and 9.6 ml (120 mmol) sulfuryl chloride is added dropwise at room temperature. Thereafter the reaction mixture is kept at 60 °C for three hours. Subsequently the reaction mixture is cooled, poured onto ice and extracted with diethylether. The ethereal layer is extracted twice with 10 percent by weight sodium hydroxide solution, dried over sodium sulphate and the solvent is evaporated. The thus obtained pale yellow oil is triturated with hexane, the white crystalline product is stirred in hexane, washed with hexane, filtered, dried and used without further purification. An analytical sample can be obtained by recrystallization from hexane.

Yield, 10.90 g (85 %) white powder

Melting point, 65-67 °C (hexane)

IR (KBr): 3165, 2964, 1713 (C=O), 1455 cm⁻¹.

¹H-NMR (CDCl₃, TMS, 400 MHz): 8.38 (br s, 1H, NH), 7.20 (d, 1H, *J* = 1.9 Hz, H-6), 6.97 (d, 1H, *J* = 1.8 Hz, H-4), 3.38 (t, 2H, *J* = 6.7 Hz, CH₂Cl), 1.95-1.84 (m, 2H, CH₂), 1.76-1.60 (m, 4H, 2 x CH₂), 1.19-1.16 (m, 1H), 1.04-0.96 (m, 1H), 0.62 (t, 3H, *J* = 7.4 Hz, CH₃) ppm.

¹³C-NMR (CDCl₃, TMS, 101 MHz): 180.5, 137.7, 135.1, 128.3, 127.6, 121.9, 115.7, 55.7, 44.3, 36.8, 32.5, 31.0, 21.7, 8.5 ppm.

Elemental Analysis [calculated on the basis of the Formula C₁₄H₁₆Cl₃NO (320.65)]
Calculated: C 52.44, H 5.03, N 4.37, Cl 33.17%
Measured: C 52.37, H 4.97, N 4.27, Cl 33.18%

### Example 3

### 5,7-Dichloro-3-{4-[4-(4-chlorophenyl)-piperazin-1-yl]butyl}-3-ethyl-1,3-dihydro-2H-indol-2-one

1-(4-chlorophenyl)-piperazine (2.36 g; 12 mmol) is heated under slow stirring to 180 °C and at this temperature, 5,7-dichloro-3-(4-chlorobutyl)-3-ethyl-1,3-dihydro-2*H*-indol-2-one (3.85 g, 12 mmol) and sodium carbonate (1.36 g; 12 mmol) are added. After one hour, the melt is allowed to cool, ethylacetate and water are added and the layers are separated. The organic phase is evaporated and the thus obtained oil is subjected to column chromatographic purification using Kieselgel 60 stationary phase and ethylacetate as eluent. The main fraction contains the oily product, which is crystallized by triturating with diethylether, filtered and recrystallized from the mixture of heptane-ethylacetate.

Yield, 3.46 g (60 %), white powder

Melting point, 152-154 °C (heptane-ethylacetate)

IR (KBr): 3137, 1719 (C=O), 826 cm⁻¹.

¹H-NMR (CDCl₃, TMS, 400 MHz): 0.65 (3H, t, *J* = 7.4 Hz), 0.98-0.86 (1H, m), 1.16-1.04 (1H, m), 1.50-1.36 (2H, m), 1.80-1.70 (2H, m), 1.98-1.88 (2H, m), 2.27 (2H, t, *J* = 7.8 Hz), 2.51 (4H, t, *J* = 5.0 Hz), 3.12 (4H, t, *J =* 5.0 Hz), 6.81 (2H, d, *J* = 9.1 Hz), 7.01 (1H, d, *J* = 1.8 Hz), 7.18 (2H, d, *J* = 9.1 Hz), 7.23 (1H, d, *J* = 1.8 Hz), 8.15 (1H, s) ppm.

¹³C-NMR (CDCl₃, TMS, 101 MHz): 180.3, 149.9, 137.7, 135.3, 128.9, 128.2, 127.5, 124.4, 122.0, 117.1, 115.7, 57.9, 55.8, 53.0, 49.1, 37.5, 31.1, 26.7, 22.2, 8.5 ppm.

Elemental Analysis [calculated on the basis of the Formula C₂₄H₂₈Cl₃N₃O (480.87)]
Calculated: C 59.95, H 5.87, Cl 22.12, N 8.74 %.
Measured: C 59.80, H 5.86, Cl 21.83, N 8.72 %.

### Example 4

### Preparation of (+)-5,7-Dichloro-3-{4-[4-(4-chlorophenyl)-piperazin-1-yl]butyl}-3-ethyl-1,3-dihydro-2H-indol-2-one by resolving of the racemate

5.00 g (10.40 mmol) racemic 5,7-dichloro-3-{4-[4-(4-chlorophenyl)-piperazin-1-yl]-butyl}-3-ethyl-1,3-dihydro-2*H*-indol-2-one base are suspended in 75 ml of ethanol and 4.22 g (10.93 mmol) of (*R*,*R*)-di-p-toluyl-tartaric acid are added in portions, while the suspension becomes gradually clear. After complete dissolution, 30 ml of water are added and the mixture is stirred for two hours. After stirring, the precipitated white substance is filtered off, washed with 10 ml portions of the solvent ethanol-water 5:2 (v/v) three times and dried.

Yield, 2.60 g (57.6 %) white powder (diastereomeric salt)

Melting point, 140-142 °C

[α]₃₆₅ (c=1, MeOH) = +226.1°

¹H-NMR (CDCl₃, TMS, 500 MHz): 8.35 (s, 1H), 7.89 (d, 4H, J = 8.2 Hz), 7.20 (d, 1H, J = 1.8 Hz), 7.12 (d, 2H, J = 8.7 Hz), 7.10 (d, 4H, J = 8.1 Hz), 7.00 (d, 1H, J = 1.8 Hz), 6.64 (d, 2H, J = 8.9 Hz), 5.80 (s, 2H), 3.19 (m, 4H), 3.04 (m, 4H), 2.72 (m, 2H), 2.34 (s, 6H), 1.87 (m, 2H), 1.70 (m, 2H), 1.59 (m, 2H), 1.01 (m, 1H), 0.91 (m, 1H), 0.61 (t, 3H).

Elemental Analysis [calculated on the basis of the Formula C₄₄H₄₆Cl₃N₃O₉ (867.23)]:
Calculated: C 60.94, H 5.35, Cl 12.26, N 4.85 %.
Measured: C 60.79, H 5.45, Cl 12.41, N 4.83 %.

The thus obtained diastereomeric salt is added in portions into the stirred solution of 1.4 ml of concentrated ammonia solution in 15 ml of distilled water and subsequently the white suspension is stirred for two hours. The mixture is filtered, washed three times with 3 ml of water each and dried.

Yield, 1.42 g (56.8 %) (+)-5,7-dichloro-3-{4-[4-(4-chlorophenyl)-piperazin-1-yl]-butyl}-3-ethyl-1,3-dihydro-2*H*-indol-2-one (enantiomeric purity > 99 %).

Melting point, 139-141 °C

[α]₃₆₅ (c=1, MeOH) = +8.1°

IR (KBr): 3137, 1719, 826 cm⁻¹.

¹H-NMR (CDCl₃, TMS, 400 MHz): 0.65 (3H, t, *J* = 7.4 Hz), 0.98-0.86 (1H, m), 1.16-1.04 (1H, m), 1.50-1.36 (2H, m), 1.80-1.70 (2H, m), 1.98-1.88 (2H, m), 2.27 (2H, t, *J* = 7.8 Hz), 2.51 (4H, t, *J* = 5.0 Hz), 3.12 (4H, t, *J* = 5.0 Hz), 6.81 (2H, d, *J* = 9.1 Hz), 7.01 (1H, d, *J* = 1.8 Hz), 7.18 (2H, d, *J* = 9.1 Hz), 7.23 (1H, d, *J* = 1.8 Hz), 8.15 (1 H, s) ppm.

¹³C-NMR (CDCl₃, TMS, 101 MHz): 180.3, 149.9, 137.7, 135.3, 128.9, 128.2, 127.5, 124.4, 122.0, 117.1, 115.7, 57.9, 55.8, 53.0, 49.1, 37.5, 31.1, 26.7, 22.2, 8.5 ppm.

Elemental Analysis [calculated on the basis of the Formula C₂₄H₂₈Cl₃N₃O (480.87)]
Calculated: C 59.95, H 5.87, Cl 22.12, N 8.74 %.
Measured: C 59.89, H 5.81, Cl 22.03, N 8.92 %.

### Example 5

### Preparation of (-)-5,7-Dichloro-3-{4-[4-(4-chlorophenyl)-piperazin-1-yl]-butyl}-3-ethyl-1,3-dihydro-2H-indol-2-one by resolving the racemate

The process described in Example 4 is carried out. The filtrate of the first filtration is evaporated until no ethanol is present in the mixture and thereafter 3 ml of concentrated ammonia solution are added. The suspension is stirred until the precipitate becomes powder-like, filtered, washed three times with 5 ml of distilled water each and dried. Yield, 3.55 g (7.38 mmol), white powder.

The base thus obtained is dissolved in 53 ml of ethanol and subsequently 2.99 g (7.75 mmol) of (*S*,*S*)-di-p-toluyl-tartaric acid and 21 ml of distilled water are added. After stirring for two hours, the precipitated solids are filtered, washed three times each with 7.5 ml of ethanol-water 5:2 (v/v) solvent mixture and dried.

Yield, 3.36 g (74.6 %) white powder (diastereomeric salt)

Melting point, 140-142 °C

[α]₃₆₅ (c=1, MeOH) = -225.6°

¹H-NMR (CDCl₃, TMS, 500 MHz): 8.35 (s, 1H), 7.89 (d, 4H, J = 8.2 Hz), 7.20 (d, 1H, J = 1.8 Hz), 7.12 (d, 2H, J = 8.7 Hz), 7.10 (d, 4H, J = 8.1 Hz), 7.00 (d, 1H, J = 1.8 Hz), 6.64 (d, 2H, J = 8.9 Hz), 5.80 (s, 2H), 3.19 (m, 4H), 3.04 (m, 4H), 2.72 (m, 2H), 2.34 (s, 6H), 1.87 (m, 2H), 1.70 (m, 2H), 1.59 (m, 2H), 1.01 (m, 1H), 0.91 (m, 1H), 0.61 (t, 3H).

Elemental Analysis [calculated on the basis of the Formula C₄₄H₄₆Cl₃N₃O₉ (867.23)]
Calculated: C 60.94, H 5.35, Cl 12.26, N 4.85 %.
Measured: C 59.59, H 5.40, Cl 12.39, N 4.88 %.

The precipitated diastereomeric salt is added in portions into the solution of 1.5 ml concentrated ammonia solution in 15 ml of water and stirred for two hours. Subsequently the mixture is filtered, washed three times with 1.5 ml of water each and dried.

Yield, 1.83 g (73.2 %) (-)-5,7-dichloro-3-{4-[4-(4-chlorophenyl)-piperazin-1-yl]butyl}-3-ethyl-1,3-dihydro-2*H*-indol-2-one (enantiomer content > 99 %)

Melting point, 139-140 °C

[α]₃₆₅ (c=1, MeOH) = -7.8°

IR (KBr): 3137, 1719 (C=O), 826 cm⁻¹.

¹H-NMR (CDCl₃, TMS, 400 MHz): 0.65 (3H, t, *J* = 7.4 Hz), 0.98-0.86 (1H, m), 1.16-1.04 (1H, m), 1.50-1.36 (2H, m), 1.80-1.70 (2H, m), 1.98-1.88 (2H, m), 2.27 (2H, t, *J* = 7.8 Hz), 2.51 (4H, t, *J* = 5.0 Hz), 3.12 (4H, t, *J* = 5.0 Hz), 6.81 (2H, d, *J* = 9.1 Hz), 7.01 (1H, d, *J* = 1.8 Hz), 7.18 (2H, d, *J* = 9.1 Hz), 7.23 (1H, d, *J* = 1.8 Hz), 8.15 (1H, s) ppm.

¹³C-NMR (CDCl₃, TMS, 101 MHz): 180.3, 149.9, 137.7, 135.3, 128.9, 128.2, 127.5, 124.4, 122.0, 117.1, 115.7, 57.9, 55.8, 53.0, 49.1, 37.5, 31.1, 26.7, 22.2, 8.5 ppm.

Elemental Analysis [calculated on the basis of the Formula C₂₄H₂₈Cl₃N₃O (480.87)]
Calculated: C 59.95, H 5.87, Cl 22.12, N 8.74 %.
Measured: C 60.08, H 5.80, Cl 21.89, N 8.98 %.

## Claims

1. Enantiomers of 5,7-dichloro-3-{4-[4-(4-chlorophenyl)-piperazin-1-yl]butyl}-3-ethyl-1,3-dihydro-2H-indol-2-one of the Formula (II), 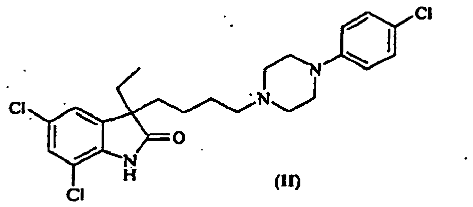 and pharmaceutically acceptable salts thereof.

2. (+)-,5,7-dichloro-3-{4-[4-(4-chlorophenyl)-piperazin-1-yl]butyl}-3-ethyl-1,3-dihydro-2H-indol-2-one of the Formula (I) 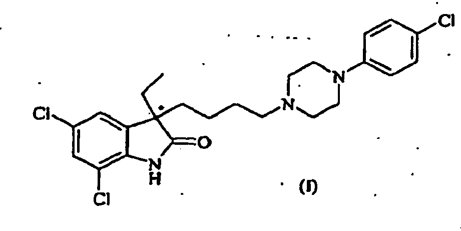 and pharmaceutically acceptable salts thereof according to claim 1.

3. (-)-5,7-dichloro-3-{4-[4-(4-chlorophenyl)-piperazin-1-yl]butyl}-3-ethyl-1,3-dihydro-2H-indol-2-one of the Formula (I) 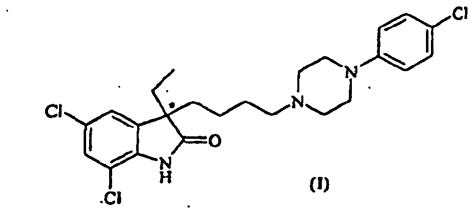 and pharmaceutically acceptable salts thereof according to claim 1.

4. Medicaments containing (+)-5,7-dichloro-3-{4-[4-(4-chlorophenyl)-piperazin-1-yl]butyl)-3-ethyl-1,3-dihydro-2H-indol-2-one of the Formula (I) or (-)-5,7-dichloro-3-{4-[4-(4-chlorophenyl)-piperazin-1-yl]butyl}-3-ethyl-1,3-dihydro-2H-indol-2-one of the Formula (I) or a pharmaceutically acceptable salt thereof according to claims 2 and 3 as active ingredient.

5. Medicaments according to claim 4 for use in the treatment or prevention of the diseases of the central nervous system including depression, anxiety, schizofrenia, mood disturbance, social phobia, agoraphobia, other specific phobias, mania, mental decline, cognitive disorder, sexual disorders of central nervous system origin, memory disturbance following trauma, posttraumatic stress disorder, cerebral trauma, dementia, cell death occuring in the central nervous system, Alzheimer-disease, stress disease, gastrointestinal diseases, cardiovascular diseases, kidney insufficiency, tinnitus and loss of hearing.

6. Medicaments according to claim 4 for use in the treatment of diseases or disorders of the central nervous system associated with the dopamine D₄ receptor.

7. The use of (+)-5,7-dichloro-3-{4-[4-(4-chlorophenyl)-piperazin-1-yl]butyl}-3-ethyl-1,3-dihydro-2H-indol-2-one of the Formula (I) or (-)-5,7-dichloro-3-{4-[4-(4-chlorophenyl)-piperazin-1-yl]butyl}-3-ethyl-1,3-dihydro-2H-indol-2-one according to claims 2 and 3 for the preparation of medicaments for use in the treatment of diseases or disorders of the central nervous system associated with the dopamine D₄ receptor.

8. Process for the preparation of (+)-5,7-dichloro-3-{4-[4-(4-chlorophenyl)-piperazin-1-yl]butyl}-3-ethyl-1,3-dihydro-2H-indol-2-one of the Formula (I) or (-)-5,7-dichloro-3-{4-[4-(4-chlorophenyl)-piperazin-1-yl]butyl}-3-ethyl-1,3-dihydro-2H-indol-2-one, which comprises resolving a mixture of the (+)-enantiomer of the Formula (I) and the (-)-enantiomer of the Formula (I) with an optically active acid.

9. Process according to claim 8, **characterized in that** racemic 5,7-dichloro-3-{4-[4-(4-chlorophenyl)-piperazin-1-yl]butyl}-3-ethyl-1,3-dihydro-2H-indol-2-one of the Formula (II) is used as starting material.

10. Process according to claim 8 or claim 9, **characterized in that** as optically active acid, an optically active tartaric acid derivative, preferably optically active di-p-toluyl-tartaric acid is used.

11. Process according to any of claims 8 to 10, **characterized in that** the resolution of the enantiomers is carried out in a dipolar aprotic or protic solvent, preferably in the mixture of an aliphatic alcohol comprising 1 to 4 carbon atoms and water.

12. Process according to any of claims 8 to 11, **characterized in that** the resolution is carried out in ethanol-water solvent mixture, using optically active di-p-toluyl-tartaric acid.

13. The use of (+) - or (-)-5,7-dichloro-3-{4-[4-(4-chlorophenyl)-piperazin-1-yl]butyl)-3-ethyl-1,3-dihydro-2H-indol-2-one of the Formula (I) according to claims 2 and 3 for the preparation of a medicament.

14. Process for the preparation of a medicament for use in the treatment or prevention of diseases of the central nervous system including depression, anxiety, schizofrenia, mood disturbance, social phobia, agoraphobia, phobias resulting from specific circumstances, mania, compulsive disorder, mental decline, cerebral damage, memory disturbances following traumas, posttraumatic stress disorder, cognitive disorder, sexual disorders of central nervous system origin, dementia, cellular death occurring within the central nervous system, Alzheimer-disease, stress disorder, gastrointestinal diseases, cardiovascular diseases, kidney insufficiency, tinnitus, loss of hearing, **characterized in that** (+)-5,7-dichloro-3-{4-[4-(4-chlorophenyl)-piperazin-1-yl]butyl}-3-ethyl-1,3-dihydro-2H-indol-2-one of the Formula (I) or (-)-5,7-dichloro-3-{4-[4-(4-chlorophenyl)-piperazin-1-yl]butyl}-3-ethyl-1,3-dihydro-2H-indol-2-one of the Formula (I) or a pharmaceutically acceptable salt thereof according to claims 2 and 3 is admixed with pharmaceutically acceptable vehicles or optionally with auxiliary agents and the mixture is converted into a pharmaceutical dosage form.

15. Use of (+)-5,7-dichloro-3-{4-[4-(4-chlorophenyl)-piperazin-1-yl]butyl}-3-ethyl-1,3-dihydro-2H-indol-2-one of the Formula (I) or (-)-5,7-dichloro-3-{4-[4-(4-chlorophenyl)-piperazin-1-yl]butyl}-3-ethyl-1,3-dihydro-2H-indol-2-one of the Formula (I) according to claims 2 and 3 for the preparation of medicaments for use in the treatment or prevention of diseases of the central nervous system including depression, anxiety, schizophrenia, mood disturbance, social phobia, agoraphobia, phobias resulting from specific circumstances, mania, compulsive disorder, mental decline, cerebral damage, memory disturbances following traumas, posttraumatic stress disorder, cognitive disorder, sexual disorders of central nervous system origin, dementia, cellular death occurring within the central nervous system, Alzheimer-disease, stress disorder, gastrointestinal diseases, cardiovascular diseases, kidney insufficiency, tinnitus and loss of hearing.

16. (+) -5,7-dichloro-3-{4-[4-(4-chlorophenyl) -piperazin-1-yl]butyl}-3-ethyl-1,3-dihydro-2H-indol-2-one of the Formula (I) or (-)-5,7-dichloro-3-{4-[4-(4-chlorophenyl)-piparazin-1-yl]butyl}-3-ethyl-1,3-dihydro-2H-indol-2-one of the Formula (I) according to claims 2 and 3 for use in the treatment of diseases or disorders associated with the dopamine D₄ receptor.

## Patentansprüche

1. Enantiomere von 5,7-Dichlor-3-{4-[4-(4-chlorphenyl)-piperazin-1-yl]butyl}-3-ethyl-1,3-dihydro-2H-indol-2-on mit der Formel (II) 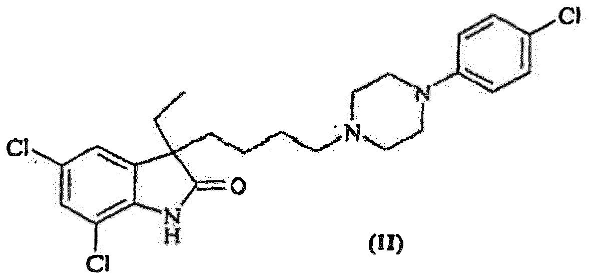 und pharmazeutisch verträgliche Salze davon.

2. (+)-5,7-Dichlor-3-{4-[4-(4-chlorphenyl)-piperazin-1-yl]butyl}-3-ethyl-1,3-dihydro-2H-indol-2-on mit der Formel (I) 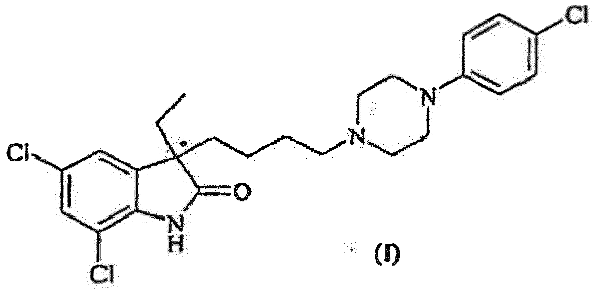 und pharmazeutisch verträgliche Salze davon nach Anspruch 1.

3. (-)-5,7-Dichlor-3-{4-[4-(4-chlorphenyl)-piperazin-1-yl]butyl}-3-ethyl-1,3-dihydro-2H-indol-2-on mit der Formel (I) 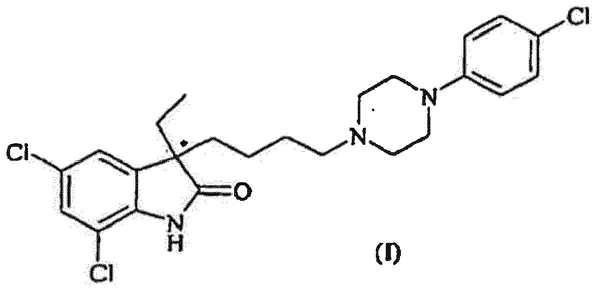 und pharmazeutisch verträgliche Salze davon nach Anspruch 1.

4. Arzneimittel, die (+)-5,7-Dichlor-3-{4-[4-(4-chlorphenyl)-piperazin-1-yl]butyl}**-**3-ethyl-1,3-dihydro-2H-indol-2-on mit der Formel (I) oder (-)-5,7-Dichlor-3-{4-[4-(4-chlorphenyl)-piperazin-1-yl]butyl}-3-ethyl-1,3-dihydro-2H-indol-2-on mit der Formel (I) oder ein pharmazeutisch verträgliches Salz davon nach den Ansprüchen 2 und 3 als wirksamen Bestandteil enthalten.

5. Arzneimittel nach Anspruch 4 zur Verwendung bei der Behandlung oder Vorbeugung von Erkrankungen des zentralen Nervensystems, einschließlich Depression, Angststörung, Schizophrenie, Stimmungsschwankungen, anderer spezifischer Phobien, Manie, geistigen Verfalls, kognitiver Störung, sexueller Störungen, die im zentralen Nervensystem entstehen, Gedächtnisstörung nach einem Trauma, posttraumatischer Belastungsstörung, zerebralen Traumas, Demenz, eines im zentralen Nervensystem auftretenden Zelltods, Alzheimer-Krankheit, Stresserkrankung, gastrointestinaler Erkrankungen, Herz-Kreislauf-Erkrankungen, Niereninsuffizienz, Tinnitus und Hörverlust.

6. Arzneimittel nach Anspruch 4 zur Verwendung bei der Behandlung von Krankheiten oder Erkrankungen des zentralen Nervensystems in Zusammenhang mit dem Dopamin D₄-Rezeptor.

7. Verwendung von (+)-5,7-Dichlor-3-{4-[4-(4-chlorphenyl)-piperazin-1-yl]butyl}-3-ethyl-1,3-dihydro-2H-indol-2-on mit der Formel (I) oder (-)-5,7-Dichlor-3-{4-[4-(4-chlorphenyl)-piperazin-1-yl]butyl}-3-ethyl-1,3-dihydro-2H-indol-2-on nach den Ansprüchen 2 und 3 zur Herstellung von Arzneimitteln zur Verwendung bei der Behandlung von Krankheiten oder Erkrankungen des zentralen Nervensystems in Zusammenhang mit dem Dopamin D₄-Rezeptor.

8. Verfahren zur Herstellung von (+)-5,7-Dichlor-3-{4-[4-(4-chlorphenyl)-piperazin-1-yl]butyl}-3-ethyl-1,3-dihydro-2H-indol-2-on mit der Formel (I) oder (-)-5,7-Dichlor-3-{4-[4-(4-chlorphenyl)-piperazin-1-yl]butyl}-3-ethyl-1,3-dihydro-2H-indol-2-on, das das Auflösen einer Mischung aus dem (+)-Enantiomer mit der Formel (I) und dem (-)-Enantiomer mit der Formel (I) mit einer optisch aktiven Säure umfasst.

9. Verfahren nach Anspruch 8, **dadurch gekennzeichnet, dass** das racemische 5,7-Dichlor-3-{4-[4-(4-chlorphenyl)-piperazin-1-yl]butyl}-3-ethyl-1,3-dihydro-2H-indol-2-on mit der Formel (II)als Ausgangsstoff verwendet wird.

10. Verfahren nach Anspruch 8 oder Anspruch 9, **dadurch gekennzeichnet, dass** als optisch aktive Säure ein optisch aktives Weinsäurederivat, vorzugsweise die optisch aktive Di-p-toluyl-weinsäure verwendet wird.

11. Verfahren nach einem der Ansprüche 8 bis 10, **dadurch gekennzeichnet, dass** das Auflösen der Enantiomere in einem dipolaren aprotischen oder protischen Lösungsmittel, vorzugsweise in der Mischung eines 1 bis 4 Kohlenstoffatome umfassenden aliphatischen Alkohols und Wasser ausgeführt wird.

12. Verfahren nach einem der Ansprüche 8 bis 11, **dadurch gekennzeichnet, dass** das Auflösen in einer Ethanol-Wasser-Lösungsmittelmischung unter Verwendung einer optisch aktiven Di-p-toluyl-weinsäure ausgeführt wird.

13. Verwendung von (+)- oder (-)-5,7-Dichlor-3-{4-[4-(4-chlorphenyl)-piperazin-1-yl]butyl}-3-ethyl-1,3-dihydro-2H-indol-2-on mit der Formel (I) nach den Ansprüchen 2 und 3 für die Herstellung eines Arzneimittels.

14. Verfahren für die Herstellung eines Arzneimittels zur Verwendung bei der Behandlung oder Vorbeugung von Erkrankungen des zentralen Nervensystems, einschließlich Depression, Angststörung, Schizophrenie, Stimmungsschwankungen, sozialer Phobie, Agoraphobie, aus spezifischen Umständen folgenden Phobien, Manie, Zwangsstörung, cerebraler Schädigung, geistigen Verfalls, Gedächtnisstörung nach Traumata, posttraumatischer Belastungsstörung, kognitiver Störung, sexueller Störungen, die im zentralen Nervensystem entstehen, Demenz, eines im zentralen Nervensystem auftretenden Zelltods, Alzheimer-Krankheit, Stresserkrankung, gastrointestinaler Erkrankungen, Herz-Kreislauf-Erkrankungen, Niereninsuffizienz, Tinnitus und Hörverlust, **dadurch gekennzeichnet, dass** (+)-5,7-Dichlor-3-{4-[4-(4-chlorphenyl)-piperazin-1-yl]butyl}-3-ethyl-1,3-dihydro-2H-indol-2-on mit der Formel (I) oder (-)-5,7-Dichlor-3-{4-[4-(4-chlorphenyl)-piperazin-1-yl]butyl}-3-ethyl-1,3-dihydro-2H-indol-2-on mit der Formel (I) oder ein pharmazeutisch verträgliches Salz davon nach den Ansprüchen 2 und 3 mit pharmazeutisch verträglichen Vehikeln oder wahlweise mit Hilfsmitteln vermengt wird und die Mischung in eine pharmazeutische Darreichungsform überführt wird.

15. Verwendung von (+)-5,7-Dichlor-3-{4-[4-(4-chlorphenyl)-piperazin-1-yl]butyl}-3-ethyl-1,3-dihydro-2H-indol-2-on mit der Formel (I) oder (-)-5,7-Dichlor-3-{4-[4-(4-chlorphenyl)-piperazin-1-yl]butyl}-3-ethyl-1,3-dihydro-2H-indol-2-on mit der Formel (I) nach den Ansprüchen 2 und 3 zur Herstellung von Arzneimitteln zur Verwendung bei der Behandlung oder Vorbeugung von Erkrankungen des zentralen Nervensystems, einschließlich Depression, Angststörung, Schizophrenie, Stimmungsschwankungen, sozialer Phobie, Agoraphobie, aus spezifischen Umständen folgenden Phobien, Manie, Zwangsstörung, geistigen Verfalls, cerebraler Schädigung, Gedächtnisstörungen nach Traumata, posttraumatischer Belastungsstörung, kognitiver Störung, sexueller Störungen, die im zentralen Nervensystem entstehen, Demenz, eines im zentralen Nervensystem auftretenden Zelltods, Alzheimer-Krankheit, Stresserkrankung, gastrointestinaler Erkrankungen, Herz-Kreislauf-Erkrankungen, Niereninsuffizienz, Tinnitus und Hörverlust.

16. (+)-5,7-Dichlor-3-{4-[4-(4-chlorphenyl)-piperazin-1-yl]butyl}-3-ethyl-1,3-dihydro-2H-indol-2-on mit der Formel (I) oder (-)-5,7-Dichlor-3-{4-[4-(4-chlorphenyl)-piperazin-1-yl]butyl}-3-ethyl-1,3-dihydro-2H-indol-2-on mit der Formel (I) nach den Ansprüchen 2 und 3 zur Verwendung bei der Behandlung von Krankheiten oder Erkrankungen des zentralen Nervensystems in Zusammenhang mit dem Dopamin D₄-Rezeptor.

## Revendications

1. Enantiomères de 5,7-dichloro-3-{4-[4-(4-chlorophényl)-pipérazin-1yl]butyl}-3-éthyl-1,3-dihydro-2H-indol-2-one de la Formule (II) 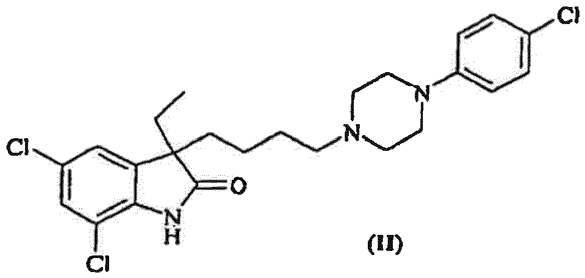 et sels pharmaceutiquement acceptables de ceux-ci.

2. (+)-5,7-dichloro-3-{4-[4-(4-chlorophényl)-pipérazin-1yl]butyl}-3-éthyl-1,3-dihydro-2H-indol-2-one de la Formule (I) 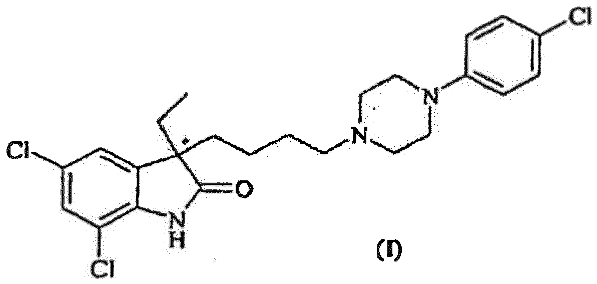 et sels pharmaceutiquement acceptables de celle-ci selon la revendication 1.

3. (-)-5,7-dichloro-3-{4-[4-(4-chlorophényl)-pipérazin-1yl]butyl}-3-éthyl-1,3-dihydro-2H-indol-2-one de la Formule (I) 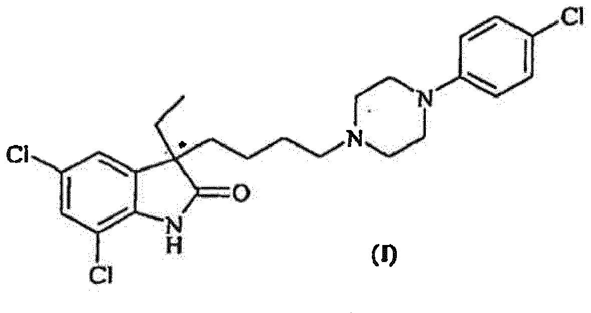 et sels pharmaceutiquement acceptables de celle-ci selon la revendication 1.

4. Médicaments contenant de la (+)-5,7-dichloro-3-{4-[4-(4-chlorophényl)-pipérazin-1yl]butyl}-3-éthyl-1,3-dihydro-2H-indol-2-one de la Formule (I) ou de la (-)-5,7-dichloro-3-{4-[4-(4-chlorophényl)-pipérazin-1yl]butyl}-3-éthyl-1,3-dihydro-2H-indol-2-one de la Formule (I) ou un sel pharmaceutiquement acceptable de celle-ci selon les revendications 2 et 3 comme ingrédient actif.

5. Médicaments selon la revendication 4 destinés à être utilisés dans le traitement ou la prévention des maladies du système nerveux central y compris la dépression, l'anxiété, la schizophrénie, des troubles de l'humeur, la phobie sociale, l'agoraphobie, d'autres phobies spécifiques, la manie, le déclin mental, un trouble cognitif, des troubles sexuels d'origine du système nerveux central, des troubles de la mémoire suite à un traumatisme, un état de stress post-traumatique, un traumatisme cérébral, la démence, la mort cellulaire se produisant dans le système nerveux central, la maladie d'Alzheimer, la maladie du stress, des maladies gastro-intestinales, des maladies cardiovasculaires, une insuffisance rénale, l'acouphène et la perte d'audition.

6. Médicaments selon la revendication 4 destinés à être utilisés dans le traitement de maladies ou de troubles du système nerveux central associés au récepteur de dopamine D₄.

7. Utilisation de (+)-5,7-dichloro-3-{4-[4-(4-chlorophényl)-pipérazin-1yl]butyl}-3-éthyl-1,3-dihydro-2H-indol-2-one de la Formule (I) ou de (-)-5,7-dichloro-3-{4-[4-(4-chlorophényl)-pipérazin-1yl]butyl}-3-éthyl-1,3-dihydro-2H-indol-2-one selon les revendications 2 et 3 pour la préparation de médicaments destinés à être utilisés dans le traitement de maladies ou de troubles du système nerveux central associés au récepteur de dopamine D₄.

8. Procédé de préparation de (+)-5,7-dichloro-3-{4-[4-(4-chlorophényl)-pipérazin-1yl]butyl}-3-éthyl-1,3-dihydro-2H-indol-2-one de la Formule (I) ou de (-)-5,7-dichloro-3-{4-[4-(4-chlorophényl)-pipérazin-1yl]butyl}-3-éthyl-1,3-dihydro-2H-indol-2-one, qui comprend la dissolution d'un mélange de l'énantiomère (+) de la Formule (I) et de l'énantiomère (-) de la Formule (I) avec un acide optiquement actif.

9. Procédé selon la revendication 8, **caractérisé en ce que** de la 5,7-dichloro-3-{4-[4-(4-chlorophényl)-pipérazin-1yl]butyl}-3-éthyl-1,3-dihydro-2H-indol-2-one racémique de la Formule (II) est utilisée comme matériau de départ.

10. Procédé selon la revendication 8 ou la revendication 9, **caractérisé en ce qu'**un dérivé d'acide tartarique optiquement actif, de préférence l'acide di-p-toluyl-tartarique optiquement actif, est utilisé en tant qu'acide optiquement actif.

11. Procédé selon l'une quelconque des revendications 8 à 10, **caractérisé en ce que** la dissolution des énantiomères est effectuée dans un solvant aprotique ou protique dipolaire, de préférence dans le mélange d'un alcool aliphatique comprenant 1 à 4 atomes de carbone et d'eau.

12. Procédé selon l'une quelconque des revendications 8 à 11, **caractérisé en ce que** la dissolution est effectuée dans un mélange de solvants éthanol-eau en utilisant de l'acide di-p-toluyl-tartarique optiquement actif.

13. Utilisation de (+)- ou (-)-5,7-dichloro-3-{4-[4-(4-chlorophényl)-pipérazin-1yl]butyl}-3-éthyl-1,3-dihydro-2H-indol-2-one de la Formule (I) selon les revendications 2 et 3 pour la préparation d'un médicament.

14. Procédé de préparation d'un médicament destiné à être utilisé dans le traitement ou la prévention de maladies du système nerveux central y compris la dépression, l'anxiété, la schizophrénie, des troubles de l'humeur, la phobie sociale, l'agoraphobie, des phobies résultant de circonstances spécifiques, la manie, un trouble compulsif, le déclin mental, un dommage cérébral, des troubles de la mémoire suite à des traumatismes, un état de stress post-traumatique, un trouble cognitif, des troubles sexuels d'origine du système nerveux central, la démence, la mort cellulaire se produisant au sein du système nerveux central, la maladie d'Alzheimer, un trouble du stress, des maladies gastro-intestinales, des maladies cardiovasculaires, une insuffisance rénale, l'acouphène, la perte d'audition, **caractérisé en ce que** de la (+)-5,7-dichloro-3-{4-[4-(4-chlorophényl)-pipérazin-1yl]butyl}-3-éthyl-1,3-dihydro-2H-indol-2-one de la Formule (I) ou de la (-)-5,7-dichloro-3-{4-[4-(4-chlorophényl)-pipérazin-1yl]butyl}-3-éthyl-1,3-dihydro-2H-indol-2-one de la Formule (I) ou un sel pharmaceutiquement acceptable de celle-ci selon les revendications 2 et 3 est mélangé(e) à des excipients pharmaceutiquement acceptables ou en option à des agents auxiliaires, et le mélange est converti en une forme de dosage pharmaceutique.

15. Utilisation de (+)-5,7-dichloro-3-{4-[4-(4-chlorophényl)-pipérazin-1yl]butyl}-3-éthyl-1,3-dihydro-2H-indol-2-one de la Formule (I) ou de (-)-5,7-dichloro-3-{4-[4-(4-chlorophényl)-pipérazin-1yl]butyl}-3-éthyl-1,3-dihydro-2H-indol-2-one de la Formule (I) selon les revendications 2 et 3 pour la préparation de médicaments destinés à être utilisés dans le traitement ou la prévention de maladies du système nerveux central y compris la dépression, l'anxiété, la schizophrénie, des troubles de l'humeur, la phobie sociale, l'agoraphobie, des phobies résultant de circonstances spécifiques, la manie, un trouble compulsif, le déclin mental, un dommage cérébral, des troubles de la mémoire suite à des traumatismes, un état de stress post-traumatique, un trouble cognitif, des troubles sexuels d'origine du système nerveux central, la démence, la mort cellulaire se produisant au sein du système nerveux central, la maladie d'Alzheimer, un trouble du stress, des maladies gastro-intestinales, des maladies cardiovasculaires, une insuffisance rénale, l'acouphène et la perte d'audition.

16. (+)-5,7-dichloro-3-{4-[4-(4-chlorophényl)-pipérazin-1yl]butyl}-3-éthyl-1,3-dihydro-2H-indol-2-one de la Formule (I) ou (-)-5,7-dichloro-3-{4-[4-(4-chlorophényl)-pipérazin-1yl]butyl}-3-éthyl-1,3-dihydro-2H-indol-2-one de la Formule (I) selon les revendications 2 et 3 destinée à être utilisée dans le traitement de maladies ou de troubles associés au récepteur de dopamine D₄.
